# EUROPEAN PATENT SPECIFICATION

(11) **EP 2 099 460 B1**
(45) Date of publication and mention of the grant of the patent: **07.09.2016**
(21) Application number: 07868944.5
(22) Date of filing: 29.11.2007
(51) Int. Cl.: A61K 31/56, C07J 1/00, A61P 35/00

(54) **6-ALKOXYALKYL ESTRADIOL DERIVATIVES AND METHODS OF USE**
6-ALKOXYALKYL-ESTRADIOL-DERIVATE UND VERFAHREN ZU IHRER VERWENDUNG
DÉRIVÉS DU 6-ALCOXYALKYLOESTRADIOL ET PROCÉDÉS D'UTILISATION

(30) Priority: 30.11.2006 US 867980 P
(43) Date of publication of application: 16.09.2009
(73) Proprietor: Endece, LLC, Mequon, WI 53092 (US)
(72) Inventor: YARGER, James, Cedarburg, WI 53012 (US)
(74) Representative: Bailey, Sam Rogerson
(86) International application number: PCT/US2007/085913
(87) International publication number: WO 2008/067450

(56) References cited:
- WO-A-2005/070951
- WO-A-2007/118832
- DD-A1- 258 820
- US-A1- 2003 055 029
- US-A1- 2006 009 434
- US-A1- 2007 088 013
- BUZZETTI, F. ET AL.: STEROIDS, vol. 58, 1993, pages 527-532, XP002567075
- SCHNEIDER F ET AL: "ÜBER DEN VERLAUF DER UMSETZUNG VON STEROID-3,5-DIENAMINEN MIT FORMALDEHYD" HELVETICA CHIMICA ACTA, VERLAG HELVETICA CHIMICA ACTA, BASEL, CH, vol. 56, no. 7, 1 January 1973 (1973-01-01), pages 2396-2404, XP002513720 ISSN: 0018-019X

## Description

### FIELD OF THE INVENTION

The present disclosure relates to compositions and methods of making 6-alkoxyalkyl Estradiol compounds with the general formula (I) and in particular of compounds (R or S) 6 hydroxymethyl- or (R or S) 6-methyloxymethyl- (8 R or S, 9S, 13 R or S, 14S, 17 R or S) -13-methyl-7,8,9,11,12,14,15,16,17-decehydrocylopenta [a] phenantherene-3, 17-diol and their pharmaceutically acceptable salts, or prodrugs thereof. The present disclosure also pertains to pharmaceutical compositions comprising such compounds, both in vitro and in vivo, for both diagnostic applications and treatment of proliferative conditions, such as cancer.

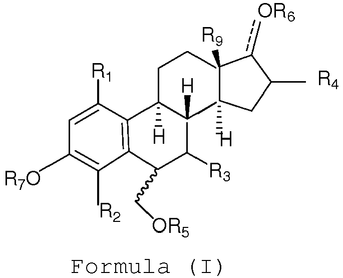

### BACKGROUND OF THE INVENTION

Proliferative cell disorders such as tumors and primary malignant tumors {herein, cancer(s)} in particular are problematic given their tendency to invade surrounding tissues and metastasize to distant organs in the body. To date, the most frequently used methods for treating neoplasia, especially solid tumor forms of neoplasia, include surgical procedures, radiation therapy, drug therapies, and combinations of the foregoing.

With over million cases of cancer being diagnosed annually, and cancer claiming more than half a million lives in the United States each year, there is increased need in new therapeutic modalities against such condition. Prostate, lung and colorectal remains the most common cancer among men; while breast, colorectal and lung cancers are the most common cancers among women.

In recent years, there have been significant gains in the management of these conditions. At least one of the success stories in the clinical management of a cancer is the early diagnosis and treatment options now available for primary breast cancer. The introduction of effective and nontoxic anti-estrogen agents that block the actions of estrogen has been shown effective.

Tamoxifen is primarily one of the first selective estrogen receptor modulators that have become first-line therapy for hormonal treatment of breast cancer, both for adjuvant treatment and for therapy of metastatic disease. Tamoxifen is a competitive inhibitor of estrodiol binding to the estrogen receptor inhibiting its estrogen binding to the estrogen binding element on DNA. In addition, there has been an increased interest in the use of aromatase inhibitors to block specifically the local production of estrogens that may contribute substantially to hormone responsive disease such as breast cancer.
Aromatase(CYP19) is described as the principal enzyme that converts androgens to estrogens both in pre- and postmenopausal women. Estrogen deprivation through aromatase inhibition is described as an effective and selective treatment for some postmenopausal patients with hormone-dependent breast cancer. Exemestane (which is sold as Aromasin, is chemically described as 6-methylenandrosta-1,4-diene-3,17-dione)and acts as an irreversible, steroidal aromatase inactivator. It is believed to act as a false substrate for the aromatase enzyme, and processed to an intermediate that binds irreversibly to the active site of the enzyme causing its inactivation. U.S. Patent Nos. 4,808,616, and 4,904,650, disclose 6-alkylidenandrosta-1,4-diene-3,17-dione derivatives, such as exemestane, and methods of making them. U.S. Patent No. 4,876,045 discloses a method of preparing 6-methylene derivatives of androsta-1,4-diene-3,17-diones. U.S. Patent No. 4,990,635 discloses a process for making 6-methylene derivatives of androsta-1,4-diene-3,17-diones.

The preparation of intermediates that may be useful in preparing exemestane is disclosed in U.S. Patent No. 3,274,176. In German patent DD 258820, 6-hydroxymethyl-androsta-1,4-diene-3,17-dione is prepared from androsta-1,4-diene-3,17-dione via 1,3-dipyrrolidinoandrosta-3,5-dien-17-one.

Co-pending international application no. PCT/US2005/001248 filed January 14, 2005 (PCT Publication Number WO 2005/070951) also describes the preparation of intermediates that are useful in preparing exemestane. The structure of Exemestane is shown below.

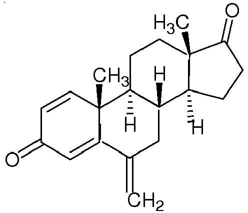

Schneider et. al, in "Course of the reaction of steroidal 3,5-dienamines with formaldehyde", Helvetica Chimica Acta (1973), 56(7), 2396-2404, discloses the following compounds: 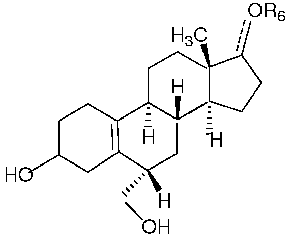 where the ---- symbol represents a double bond, it means a keto group and no R₆ is present; and where the ---- symbol represents a single bond R₆ is hydrogen (i.e. an alcohol group). Unlike the compounds of the present invention, Schneider's compounds do not embrace estrodial, testastrone or dihydrotestastrone variations.

A tri-hydroxyl substituted derivative of estranes is disclosed in U.S. Patent No. 3,377,363 to Tadanier et. al, and the 3 hydroxy substituent on the aromatic ring of the present compounds is not disclosed.

U.S. Patent No. 5,914,324 to De Funari et.al, discloses 6 hydroxy and oxy androstane derivatives for hypertension and heart failure. U.S. Patent 6,384,250 to Gobbini, et al., discloses the hydroxyl and ketone substituents at the 6 position in the preparation of (E,Z) 3-(2-aminoethoxyimino)-androstane-6,17-dione. These compounds were directed towards the treatment of heart failure. The effects of alkyl hydroxyl substitution at the 6 position is not disclosed.

Tanenbaum, et. al, "Crystallographic comparison of the estrogen and progestone receptor's ligand binding domains", Proc. Natl. Acad. Sci. USA, Biochemistry, Vol. 95(1998), pp. 5998-6003, discloses the mechanism of ER receptors and notes that estradiol containing an aromatic ring with a 3-hydroxy substituent binds well with the ER ligand binding region. It is disclosed that a flat aromatic group without the 19 methyl substituent is favored. Given the above, a need still exists to identify new and effective agents for treating cancer.

Buzzetti et al., "synthesis and aromatase inhibition by potential metabolites of exemestane (6-methylenandrosta-1,4-diene-3,17-dione)", Steroids, 1993, vol. 58, pp.527-532 describes synthesis and testing of some potential metabolites of exemestane all of which were found to be less potent in inhibiting aromatase than exemestane. The compounds all include a ketone functionality at the 3-position.

### SUMMARY OF THE INVENTION

In light of the foregoing, the present invention is directed towards chemotherapeutic compounds, compositions and their use and preparation, thereby overcoming various deficiencies and shortcomings of the prior art, including those outlined above.

An aspect of the present invention pertains to a compound of Formula I. 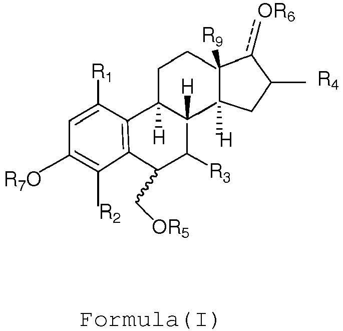
wherein R₁, R₂, R₃, R₄, R₆, R₇, and R₉ are independently hydrogen, C₁ to C₆ alkyl or substituted alkyl, halogen, sulfate, or glucuronide moieties; and the ---- symbol represents either a single or a double bond and when the-symbol is a double bond and forms a keto group at position 17, then no R₆ is present; R₅ is C₁ to C₆ alkyl or substituted alkyl;
and the 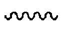 symbol represents any type of bond regardless of the stereochemistry. The compounds also embrace the enantiomers, other stereochemical isomers, hydrates, solvates, tautomers and pharmaceutically acceptable salts thereof.

The present invention relates to a compound of the invention for use in the treatment of cancer in a mammalian subject (e.g., a human patient). In this aspect of the invention, compounds are provided for inhibiting tumor or cancerous cell growth within the mammalian subject. The cells are exposed to or contacted with a compound of Formula (I) or pharmaceutically acceptable enantiomers, other stereochemical isomers, hydrates, solvates, tautomers, or salts thereof, as shown herein. In a specific, non-limiting embodiment of the present invention, a compound of Formula (I) is used to therapeutically treat an identified cancer state as described herein. In another specific non-limiting embodiment of the present invention, a composition comprising a compound of Formula (I) is used to therapeutically treat an identified cancer state as described herein.

The compounds of the present invention may be used to treat any tumor which may be either directly or indirectly effected by hormonal and/or estrogen-related activity, including but not in any way limited to solid tumors associated with breast, pancreatic, lung, colon, prostate, ovarian cancers, as well as brain, liver, spleen, kidney, lymph node, small intestine, blood cells, bone, stomach, endometrium, testicular, ovary, central nervous system, skin, head and neck, esophagus, or bone marrow cancer; as well as hematological cancers, such as leukemia, acute promyelocytic leukemia, lymphoma, multiple myeloma, myelodysplasia, myeloproliferative disease, or refractory anemia.

The compounds of the present invention may also be used in combination-based therapeutic cancer treatments in a mammalian subject. Such methods may comprise administration of a compound of Formula (I) in combination with other adjunct cancer therapies, such as chemotherapy, radiotherapy, gene therapy, hormone therapy and other cancer therapies known in the art.

In the methods of treatment of the present invention, the term "administering" shall encompass the treatment of the various conditions described with the compound specifically disclosed or with a compound which may not be specifically disclosed, but which converts to the specified compound *in vivo* after administration to the patient and exhibits therapeutic activity.

Also described is the inhibition of growth of cancer cells comprising providing to a patient a prodrug of Formula (III) wherein R₅ is a methyl or hydrogen; 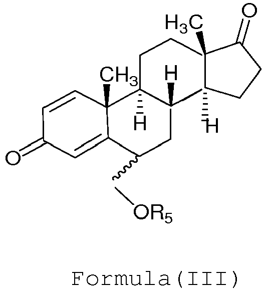 and forming metabolites of formula (IV), (V), (VI), (VII), and (VIII) wherein any of R₃, R₄, R₅, R₇, R₈ may be methyl or hydrogen. Such metabolites could include for example the structures shown below:

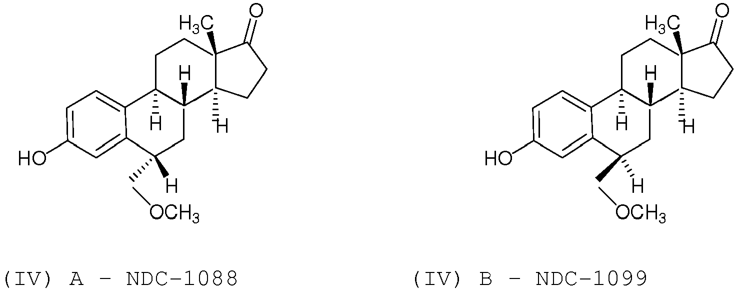

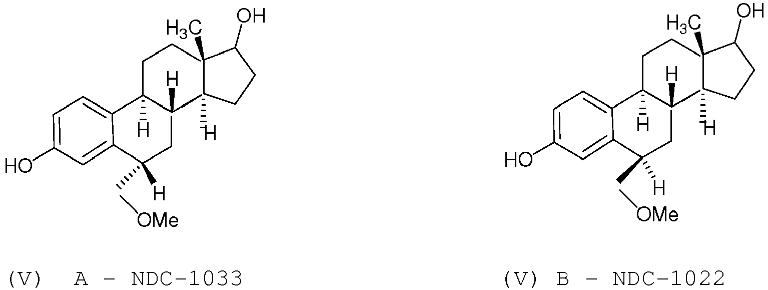

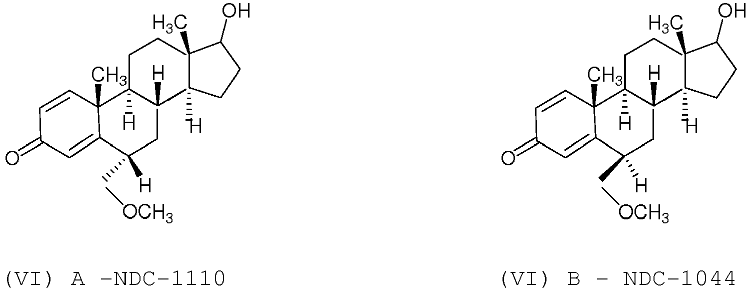

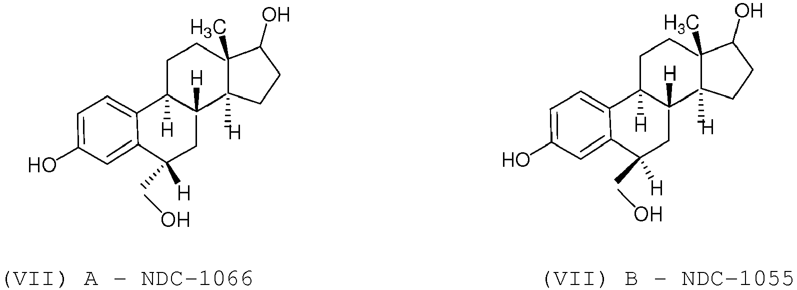

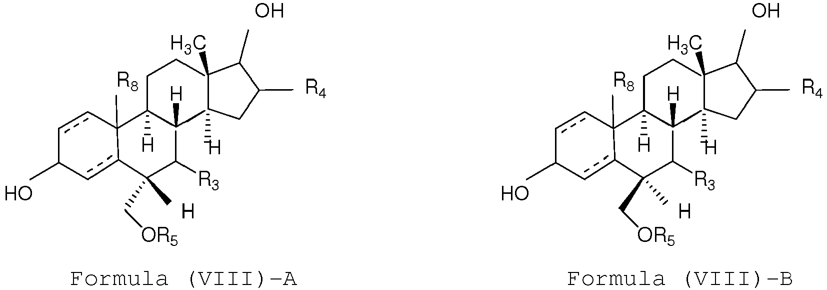

Other objects, features, benefits and advantages of the present invention will be apparent from this summary and the following descriptions of certain embodiments, and will be readily apparent to those skilled in the art having knowledge of various chemotherapeutic compounds, methods and/or modes of operation.

### BRIEF DESCRIPTION OF THE DRAWINGS

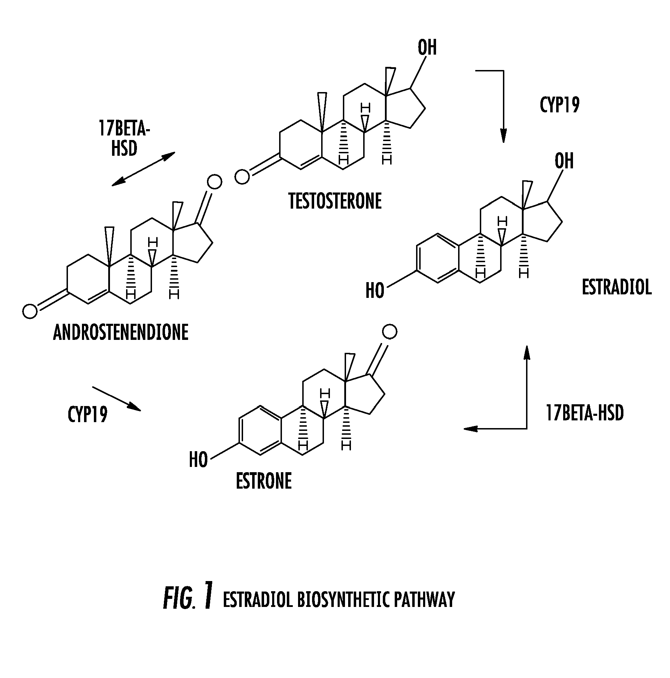

Figure 1- shows the Estradiol biosynthetic pathway.
Figure 2- shows a predicted metabolic pathway for the present compounds.

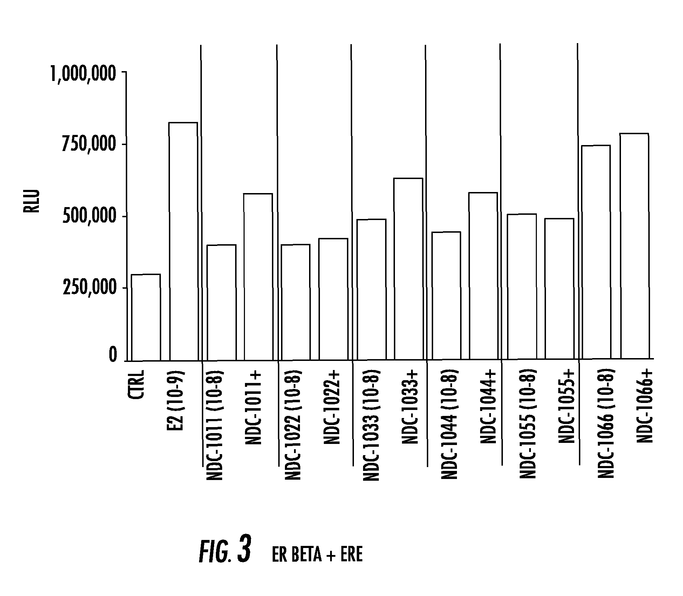

Figure 3- shows the effect of NDC-1011, NDC-1022, NDC-1033, NDC-1044, NDC-1055 and NDC-1066 on estrogen receptor beta (ER-β) activity as measured by luciferase expression (RLU = relative light units). CV-1 cells were transfected with two plasmid constructs, the reporter construct ERE-tk-luciferase and a CMV-ER-β contruct. Transfected control (Ctrl) CV-1 cells received no treatment while estradiol treated cells (E2) received estradiol added alone at 10⁻⁹ M (1 nM). In the case of NDC compounds, each compound respectively was either added alone at 10⁻⁸ M (10 nM) (as evident in the left column for each test compound) or at 10⁻⁸ M plus 10⁻⁹ M estradiol (E2) (as evident in the right column for each test compound).

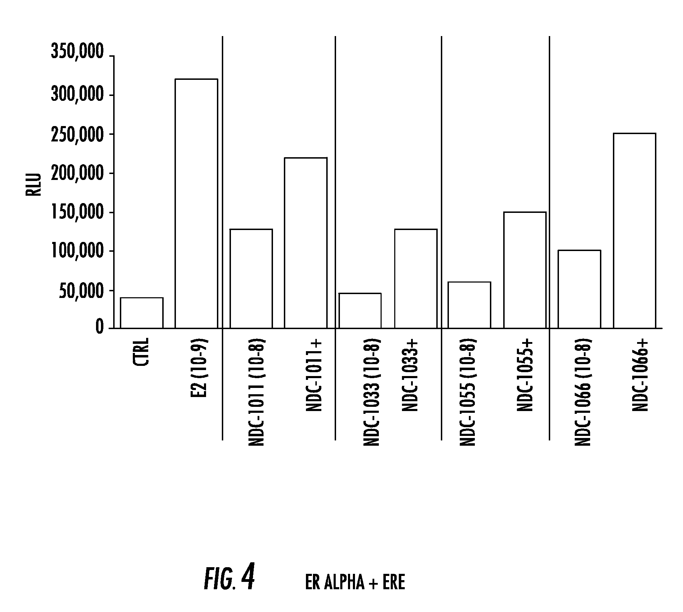

Figure 4 shows the effect of NDC-1011, NDC-1033, NDC-1055 and NDC-1066 on estrogen receptor alpha (ER-α) activity as measured by luciferase expression (RLU = relative light units). CV-1 cells were transfected with two plasmid constructs, the reporter construct ERE-tk-luciferase and a CMV-ER-α contruct. Transfected control (Ctrl) CV-1 cells received no treatment while estradiol (E2) was added alone at 10⁻⁹ M (1 nM). In the case of NDC compounds, each compound respectively was either added alone at 10⁻⁸ M (10 nM) (as evident in the left column for each test compound) or at 10⁻⁸ M plus 10⁻⁹ M estradiol (E2) (as evident in the right column for each test compound).
Figure 5 shows IC₅₀ growth inhibition data (in µM) for NDC-1022 (left columns), NDC-1033 (middle columns) and NDC-1044 (right columns) as determined in each of the cell lines HT-29, SK-OV-3, NCI-H23, MCF-7, MDA-MB-231, OVCAR-3, CAPAN-1, CAPAN-2, SH-SY5Y, A-549 and PC-3.
Figure 6 shows numerical IC₅₀ growth inhibition data (in µM) for NDC-1022, NDC-1033 and NDC-1044 as determined in each of the cell lines HT-29, SK-OV-3, NCI-H23, MCF-7, MDA-MB-231, OVCAR-3, CAPAN-1, CAPAN-2, SH-SY5Y, A-549 and PC-3.

### DETAILED DESCRIPTION OF THE INVENTION

Unless defined otherwise, all technical and scientific terms used herein have the same meaning as is commonly understood by one of skill in the art to which this invention belongs and shall be understood to have the meanings described below. Unless otherwise specified, a reference to a particular compound includes all such isomeric forms, including racemic and other mixtures thereof. Unless otherwise specified, a reference to a particular compound also includes ionic, salt, solvate (e.g., hydrate), protected forms, prodrugs, and other stereoisomers thereof, for example, as discussed herein.

It may be convenient or desirable to prepare, purify, and/or handle a corresponding salt of the active compound, for example, a pharmaceutically-acceptable salt. Examples of pharmaceutically acceptable salts are discussed in Berge et al., 1977, "Pharmaceutically Acceptable Salts," J. Pharm. Sci., Vol. 66, pp. 1-19, and discussed herein.

Anti-proliferative compounds of the present invention have application in the treatment of cancer, and so the present invention further provides anti-cancer agents. The term "anti-cancer agent" as used herein, pertains to a compound which treats a cancer (i.e., a compound which is useful in the treatment of a cancer). The anti-cancer effect may arise through one or more mechanisms, including but not limited to, the regulation of cell proliferation, the inhibition of angiogenesis (the formation of new blood vessels), the inhibition of metastasis (the spread of a tumor from its origin), the inhibition of invasion (the spread of tumor cells into neighboring normal structures), or the promotion of apoptosis (programmed cell death).

The invention further provides active compounds for use in treatment of the human or animal body by therapy. Such treatment may comprise administering to such a subject a therapeutically-effective amount of an active compound, preferably in the form of a pharmaceutical composition as discussed further herein.

The term "treatment," or "therapy" as used herein in the context of treating a condition, pertains generally to treatment and therapy of a mammalian subject, whether of a human or a non-human animal (e.g., in veterinary applications), in which some desired therapeutic effect is achieved, for example, the inhibition of the progress of the condition, and includes a reduction in the rate of progress, a halt in the rate of progress, amelioration of the condition, and/or cure of the condition. Treatment as a prophylactic measure is also included. Treatment includes combination treatments and therapies, in which two or more treatments or therapies are combined, for example, sequentially or simultaneously. Examples of treatments and therapies include, but are not limited to, chemotherapy (the administration of active agents, including, e.g., drugs, antibodies (e.g., as in immunotherapy), prodrugs (e.g., employing protecting groups including phosphoric acid derivatives and phosphinates at suitable positions such as position 3 or 17, other compounds used for photodynamic therapy, GDEPT, ADEPT, etc.); surgery; radiation therapy; and gene therapy.

The term "stereochemical isomer" as used herein, refers to isomers that differ from each other only in the way the atoms are oriented in space. The two stereoisomers particularly of importance in the instant invention are enantiomers and diastereomers depending on whether or not the two isomers are mirror images of each other. In the preferred embodiment, the claimed formulations comprise such compounds that isolated, resolved and are "substantially free of other isomers."

The term "therapeutically-effective amount," as used herein, pertains to that amount of an active compound, or a material, composition or dosage form comprising an active compound, which is effective for producing some desired therapeutic effect, commensurate with a reasonable benefit/risk ratio.

The term "patient" refers to animals, including mammals, preferably humans.

The term "region of a patient" refers to a particular area or portion of the patient afflicted with a proliferative disorder, cancer or tumor and in some instances to regions throughout the entire patient. Exemplary of such regions are the pulmonary region, the gastrointestinal region, the breast region, the renal region as well as other bodily regions, tissues, lymphocytes, receptors, organs and the like, including the vasculature and circulatory system, and cancerous tissue. "region of a patient" includes, for example, regions to be treated with the disclosed compounds and compositions. The "region of a patient" is preferably internal, although it may be external.

The term "tissue" refers generally to specialized cells which may perform a particular function. The term "tissue" may refer to an individual cell or a plurality or aggregate of cells, for example, membranes, blood or organs. The term "tissue" also includes reference to an abnormal cell or a plurality of abnormal cells. Exemplary tissues include breast tissue, including breast cells, membranous tissues, including endothelium and epithelium, laminae, connective tissue, including interstitial tissue, and tumors.

### Compounds

The present invention is directed to a chemotherapeutic compound of Formula (I): 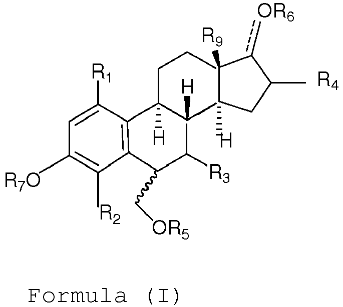
wherein R₁, R₂, R₃, R₄, R₆, R₇, and R₉ are independently hydrogen, C₁ to C₆ alkyl or substituted alkyl, halogen, sulfate, or glucuronide moieties; and the ---- symbol represents either a single or a double bond and when the-- symbol is a double bond and forms a keto group at position 17, then no R₆ is present; R₅ is C₁ to C₆ alkyl or substituted alkyl;
and the 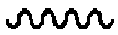 symbol represents any type of bond regardless of the stereochemistry. The compounds also embrace the enantiomers, other stereochemical isomers, hydrates, solvates, tautomers and pharmaceutically acceptable salts thereof.

In an embodiment of Formula I, R₅, R₆, R₇, R₉ are hydrogen atoms, methyl or Cl; R₁, R₂ is hydrogen or methyl; R₃, R₄ is C₁ to about C₆ alkyl or substituted alkyl, halogen andis a single bond corresponding to the alcohol group. In an another embodiment the stereochemistry at the C-6 carbon comprises a S or R enantiomer or diastereomers.

Embodiment compounds of the present invention can be used in a pharmaceutical composition. Such a composition can comprise one or more compounds selected from those discussed above, illustrated below or otherwise inferred herein, and combinations thereof. In certain embodiments, such a composition can comprise a pharmaceutically-acceptable carrier component. Without limitation, such a composition can comprise a racemic mixture of compounds. In certain embodiments, such a compound can be present as the S and R enantiomer, preferably their isolated and purified form which is substantially free of other isomers, and R₅, or R₇ can be selected from H, C₁ to C₆ alkyl or substituted alkyl, and a halogen.

The compounds of the present invention may have asymmetric centers and may occur as racemate, racemic mixture or as individual diastereomers or enantiomers such as (S)6-methyloxymethyl(8S, 9S, 13S, 14S, 17S) -13-methyl-7,8,9,11,12,14,15,16,17 -decehydrocylopenta [a] phenantherene-3, 17-diol; (R)6-methyloxymethyl(8S, 9S, 13S, 14S, 17R) -13-methyl- 7,8,9,11,12,14,15,16,17-decehydrocylopenta [a] phenantherene-3, 17-diol; (R)6-methyloxymethyl(8R, 9S, 13R, 14S, 17R) -13-methyl-7,8,9,11,12,14,15,16,17-decehydrocylopenta [a] phenantherene-3, 17-diol (NDC-1022); or (S)6-methyloxymethyl(8R, 9S, 13R, 14S, 17R) -13-methyl-7,8,9,11,12,14,15,16,17-decehydrocylopenta [a] phenantherene-3, 17-diol (NDC-1033).

Also described herein is the
preparation of the R or S enantiomers, R or S diastereomers of 6 substituted estradiols. Methods for the preparation (e.g., asymmetric synthesis) and separation (e.g., fractional crystallization and chromatographic means) of such isomeric forms are either generally known in the art or are readily obtained by adapting the methods taught herein. One such methodologies are described in the co-pending U.S. application SN 11/541,987.

Also described herein is a method for preparing a 6-hydroxymethyl or 6-methyloxymethyl derivative of estradiol. A reaction scheme for preparing estradiol derivatives is given below, Scheme 1. Such a method can comprise reaction of t-butyldimethylsilyl derivative of estradiol with LIDAKOR/THF/formaldehyde to obtain a 6-hydroxylated compound followed by such steps as:
(i) hydrolysis to obtain 6-hydroxymethyl derivative of estradiol, NDC-1066; and/or (ii)treatment with dimethylsulfate followed by hydrolysis to obtain 6-methyloxymethyl derivative of estradiol, NDC-1033. NDC-1088 can be obtained by further oxidation of NDC-1033 at the C-17 hydroxyl position.

In an alternative approach, the compounds of the present invention can also be prepared by a method comprising such steps as: (i) protecting an estrodial compound, (ii) acylating the protected estradiol compound at the benzylic 6-position with LIDAKOR/ButylLithium/Diisopropylamine/potassium tert-amylate, (iii) reducing the position 6 aldehyde with lithium aluminum hydride, (iv) deprotecting the protected regions of the estrodial compound. A reaction scheme for preparing estradiol derivatives is given below in Scheme 2.

The compounds of the present invention can be synthesized by the following methods as depicted in the schemes below:

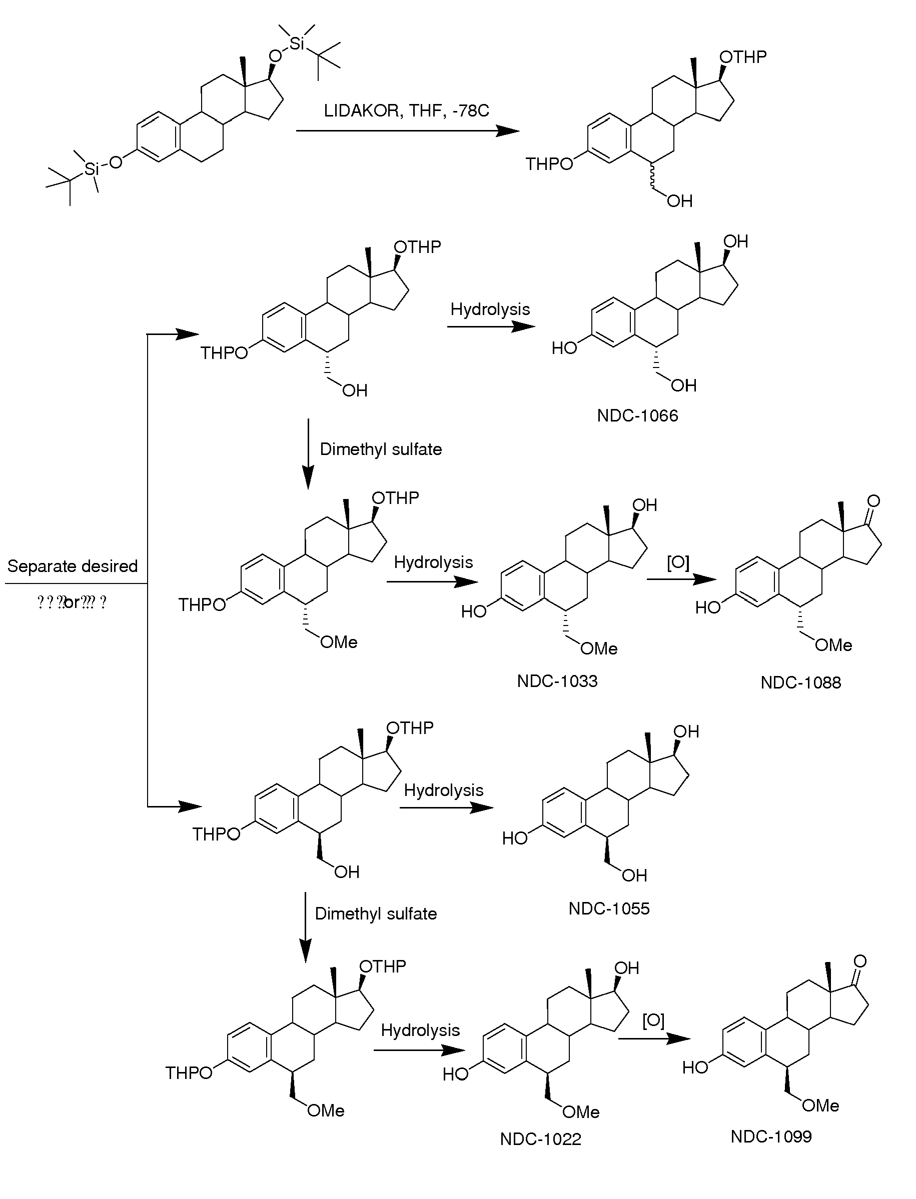

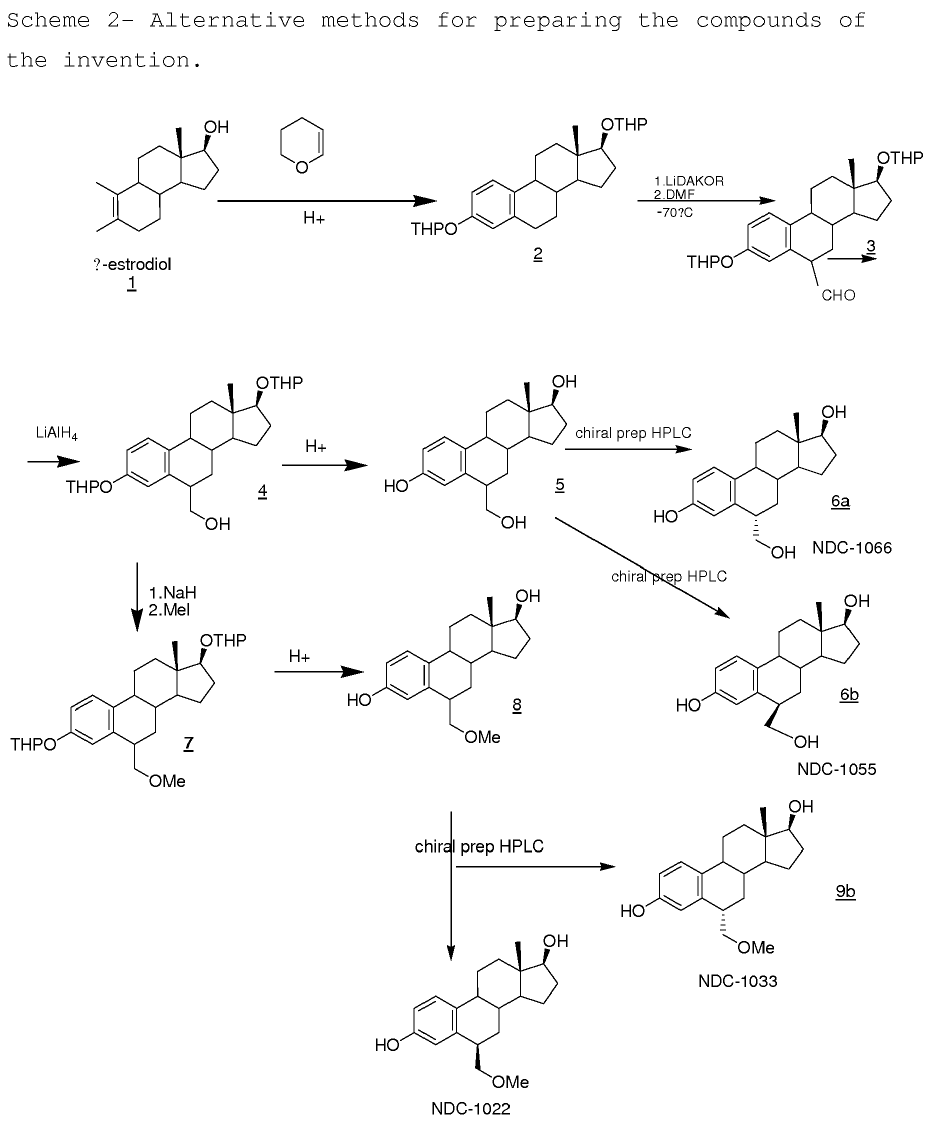

Various methyloxyalkyl derivatives, in accordance with this invention, are limited only by choice of alkylating agent, such derivatives as would be understood by those skilled in art made aware of this invention, as available through synthetic procedures of the sort described herein or straight-forward modifications thereof, such modifications as would also be understood by those skilled in the art. Accordingly, without limitation, various C₁ to C₆ alkyl and substituted alkyl (e.g., C₁ to C₆ linear, substituted linear, branched and substituted branched alkyl, such substituents as would be understood in the art) reagents can be used as described herein to prepare the corresponding methyloxyalkyl derivatives.

The present invention relates to a compound of the invention for use in the treatment of cancer in a mammalian subject (e.g., a human patient). In this aspect of the invention, compounds are provided for inhibiting tumor or cancerous cell growth. In such aspects, the cells are exposed to or contacted with a compound of Formula (I) or pharmaceutically acceptable salts or hydrates thereof: 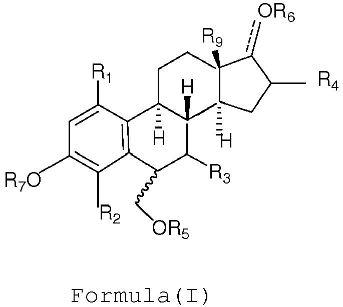
wherein R₁, R₂, R₃, R₄, R₆, R₇, and R₉ are independently hydrogen, C₁ to C₆ alkyl or substituted alkyl, halogen, sulfate, or glucuronide moieties; and the ---- symbol represents either a single or a double bond and when the-- symbol is a double bond and forms a keto group at position 17, then no R₆ is present; R₅ is C₁ to C₆ alkyl or substituted alkyl;
and the 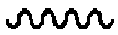 symbol represents any type of bond regardless of the stereochemistry. The compounds also embrace the enantiomers, other stereochemical isomers, hydrates, solvates, tautomers and pharmaceutically acceptable salts thereof.

These compounds may be used to treat any tumor which may be either directly or indirectly effected by hormonal and/or estrogen-related activity, including but not in any way limited to solid tumors associated with breast, pancreatic, lung, colon, prostate, ovarian cancers, as well as brain, liver, spleen, kidney, lymph node, small intestine, blood cells, bone, stomach, endometrium, testicular, ovary, central nervous system, skin, head and neck, esophagus, or bone marrow cancer; as well as hematological cancers, such as leukemia, acute promyelocytic leukemia, lymphoma, multiple myeloma, myelodysplasia, myeloproliferative disease, or refractory anemia.

In addition, administration of the compounds of the present invention for treatment of various cancer states may comprise administration of a compound of Formula (I) in combination with other adjunct cancer therapies, such as chemotherapy, radiotherapy, gene therapy, hormone therapy and other cancer therapies known in the art. Combinations of the presently disclosed compounds with other anti-cancer or chemotherapeutic agents are within the scope of the invention. Examples of such agents can be found in Cancer Principles and Practice of Oncology by V. T. Devita and S. Hellman (editors), 6th edition (Feb. 15, 2001), Lippincott Williams & Wilkins Publishers. A physician, veterinarian or clinician of ordinary skill in the art would be able to discern which combinations of agents would be useful based on the particular characteristics of the drugs and the cancer involved. Such anti-cancer agents include the following: estrogen receptor modulators, androgen receptor modulators, retinoid receptor modulators, cytotoxic agents, anti-proliferative agents, prenyl-protein transferase inhibitors, HMG-CoA reductase inhibitors, EHV protease inhibitors, reverse transcriptase inhibitors, aromatase inhibitors, and angiogenesis inhibitors.

Compounds of the present disclosure are illustrated in table I below:

**Table I**

| | | | | | | | | | |
|---|---|---|---|---|---|---|---|---|---|
| 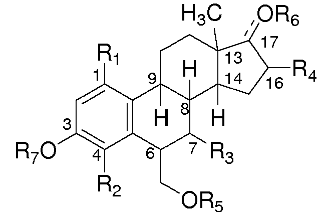 | | | | | | | | | |

| R₁, R₂, R₃, R₄: independently H, C₁-C₆ alkyl, substituted alkyl, or halogen | | | | | | | | | |
|---|---|---|---|---|---|---|---|---|---|
| R₇: H, C₁-C₆ alkyl, substituted alkyl, sulfate, or glucuronide | | | | | | | | | |
| R₆: H, C₁-C₆ alkyl, or substituted alkyl, sulfate, or glucuronide, when --- is a single bound; not present, when --- is a double bond R₅ is C₁ to C₆ alkyl or substituted alkyl; | | | | | | | | | |
| | **Substituents** | | | **Spatial Configuration** | | | | | |
| **Entry** | **R₅** | **R₆** | **R₇** | **C-6** | **C-8** | **C-9** | **C-13** | **C-14** | **C-17** |
| 1 | H | H | H | S | S | S | S | S | S |
| 2 | H | H | H | S | R | R | R | R | R |
| 3 | H | H | H | S | S | S | S | S | R |
| 4 | H | H | H | S | R | R | R | R | S |
| 5 | H | - | H | S | S | S | S | S | C=O |
| 6 | H | - | H | S | R | R | R | R | C=O |
| 7 | H | H | H | R | R | R | R | R | R |
| 8 | H | H | H | R | S | S | S | S | S |
| 9 | H | H | H | R | S | S | S | S | R |
| 10 | H | H | H | R | R | R | R | R | S |
| 11 | H | - | H | R | S | S | S | S | C=O |
| 12 | H | - | H | R | R | R | R | R | C=O |
| 13 | Me | H | H | S | S | S | S | S | S |
| 14 | Me | H | H | S | R | R | R | R | R |
| 15* | Me | H | H | S | R | S | R | S | R |
| 16 | Me | H | H | S | R | R | R | R | S |
| 17 | Me | - | H | S | S | S | S | S | C=O |
| 18 | Me | - | H | S | R | R | R | R | C=O |
| 19 | Me | H | H | R | R | R | R | R | R |
| 20** | Me | H | H | R | R | S | R | S | R |
| 21 | Me | H | H | R | S | S | S | S | R |
| 22 | Me | H | H | R | R | R | R | R | S |
| 23 | Me | - | H | R | S | S | S | S | C=O |
| 24 | Me | - | H | R | R | R | R | R | C=O |
| 25 | H | H | SO₃H | S | S | S | S | S | S |
| 26 | H | H | SO₃H | S | R | R | R | R | R |
| 27 | H | H | SO₃H | S | S | S | S | S | R |
| 28 | H | H | SO₃H | S | R | R | R | R | S |
| 29 | H | - | SO₃H | S | S | S | S | S | C=O |
| 30 | H | - | SO₃H | S | R | R | R | R | C=O |
| 31 | H | H | SO₃H | R | R | R | R | R | R |
| 32 | H | H | SO₃H | R | S | S | S | S | S |
| 33 | H | H | SO₃H | R | S | S | S | S | R |
| 34 | H | H | SO₃H | R | R | R | R | R | S |
| 35 | H | - | SO₃H | R | S | S | S | S | C=O |
| 36 | H | - | SO₃H | R | R | R | R | R | C=O |
| 37 | Me | H | SO₃H | S | S | S | S | S | S |
| 38 | Me | H | SO₃H | S | R | R | R | R | R |
| 39 | Me | H | SO₃H | S | S | S | S | S | R |
| 40 | Me | H | SO₃H | S | R | R | R | R | S |
| 41 | Me | - | SO₃H | S | S | S | S | S | C=O |
| 42 | Me | - | SO₃H | S | R | R | R | R | C=O |
| 43 | Me | H | SO₃H | R | R | R | R | R | R |
| 44 | Me | H | SO₃H | R | S | S | S | S | S |
| 45 | Me | H | SO₃H | R | S | S | S | S | R |
| 46 | Me | H | SO₃H | R | R | R | R | R | S |
| 47 | Me | - | SO₃H | R | S | S | S | S | C=O |
| 48 | Me | - | SO₃H | R | R | R | R | R | C=O |
| 49 | H | H | glucuronide | S | S | S | S | S | S |
| 50 | H | H | glucuronide | S | R | R | R | R | R |
| 51 | H | H | glucuronide | S | S | S | S | S | R |
| 52 | H | H | glucuronide | S | R | R | R | R | S |
| 53 | H | - | glucuronide | S | S | S | S | S | C=O |
| 54 | H | - | glucuronide | S | R | R | R | R | C=O |
| 55 | H | H | glucuronide | R | R | R | R | R | R |
| 56 | H | H | glucuronide | R | S | S | S | S | S |
| 57 | H | H | glucuronide | R | S | S | S | S | R |
| 58 | H | H | glucuronide | R | R | R | R | R | S |
| 59 | H | - | glucuronide | R | S | S | S | S | C=O |
| 60 | H | - | glucuronide | R | R | R | R | R | C=O |
| 61 | Me | H | glucuronide | S | S | S | S | S | S |
| 62 | Me | H | glucuronide | S | R | R | R | R | R |
| 63 | Me | H | glucuronide | S | S | S | S | S | R |
| 64 | Me | H | glucuronide | S | R | R | R | R | S |
| 65 | Me | - | glucuronide | S | S | S | S | S | C=O |
| 66 | Me | - | glucuronide | S | R | R | R | R | C=O |
| 67 | Me | H | glucuronide | R | R | R | R | R | R |
| 68 | Me | H | glucuronide | R | S | S | S | S | S |
| 69 | Me | H | glucuronide | R | S | S | S | S | R |
| 70 | Me | H | glucuronide | R | R | R | R | R | S |
| 71 | Me | - | glucuronide | R | S | S | S | S | C=O |
| 72 | Me | - | glucuronide | R | R | R | R | R | C=O |

| | | | | | | | | | |
|---|---|---|---|---|---|---|---|---|---|
| * NDC-1033; ** NDC-1022 | | | | | | | | | |

The preferred compounds in Table I include compounds 15 and 20. At least one aspect of the instant invention is directed to these preferred compound, and their use.

Also described are comparative compounds in table II below:

**Table II**

| | | | | | | | | | |
|---|---|---|---|---|---|---|---|---|---|
| 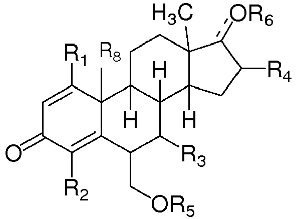 | | | | | | | | | |

| R₁, R₂, R₃, R₄: independently H, C₁-C₆ alkyl, substituted alkyl, or halogen | | | | | | | | | |
|---|---|---|---|---|---|---|---|---|---|
| R₅: H, C₁-C₆ alkyl, substituted alkyl, sulfate, or glucuronide | | | | | | | | | |
| R₆: H, C₁-C₆ alkyl, or substituted alkyl, sulfate, or glucuronide, | | | | | | | | | |
| R₈: H, C₁-C₆ alkyl, or substituted alkyl, when --- is a single bond; not present, when --- is a double bond | | | | | | | | | |
| | **Substituent** | | **Spatial Configuration** | | | | | | |
| **Entry** | **R₅** | **R₆** | **C-6** | **C-8** | **C-9** | **C-10** | **C-13** | **C-14** | **C-17** |
| 73 | H | H | S | S | S | R | S | S | S |
| 74 | H | H | S | R | R | R | R | R | R |
| 75 | H | H | S | S | S | R | S | S | R |
| 76 | H | H | S | R | R | R | R | R | S |
| 77 | H | - | S | S | S | R | S | S | C=O |
| 78 | H | - | S | R | R | R | R | R | C=O |
| 79 | H | H | R | R | R | R | R | R | R |
| 80 | H | H | R | S | S | R | S | S | S |
| 81 | H | H | R | S | S | R | S | S | R |
| 82 | H | H | R | R | R | R | R | R | S |
| 83 | H | - | R | S | S | R | S | S | C=O |
| 84 | H | - | R | R | R | R | R | R | C=O |
| 85 | Me | H | S | S | S | R | S | S | S |
| 86 | Me | H | S | R | R | R | R | R | R |
| 87 | Me | H | S | S | S | R | S | S | R |
| 88 | Me | H | S | R | R | R | R | R | S |
| 89* | Me | - | S | R | S | R | R | S | C=O |
| 90** | Me | - | R | R | S | R | R | S | C=O |
| 91 | Me | H | R | R | R | R | R | R | R |
| 92 | Me | H | S | S | S | R | S | S | S |
| 93 | Me | H | S | R | R | R | R | R | R |
| 94 | Me | H | S | S | S | R | S | S | R |
| 95 | Me | H | S | R | R | R | R | R | S |
| 96 | Me | - | R | R | S | - | R | S | C=O |
| 97 | Me | - | S | R | R | R | R | R | C=O |
| 98*** | Me | H | S | R | S | R | R | S | R |
| 99 | Me | H | R | S | S | R | S | S | S |
| 100 | Me | H | R | S | S | S | S | S | R |
| 101 | Me | H | R | R | R | S | R | R | S |
| 102 | Me | - | R | S | S | R | S | S | C=O |
| 103 | Me | - | R | R | R | R | R | R | C=O |
| 104 | H | H | S | S | S | R | S | S | S |
| 105 | H | H | S | R | R | R | R | R | R |
| 106 | H | H | S | S | S | R | S | S | R |
| 107 | H | H | S | R | R | R | R | R | S |
| 108 | H | - | S | S | S | R | S | S | C=O |
| 109 | H | - | S | R | R | R | R | R | C=O |
| 110 | H | H | R | R | R | R | R | R | R |
| 111 | H | H | R | S | S | R | S | S | S |
| 112 | H | H | R | S | S | R | S | S | R |
| 113 | H | H | R | R | R | R | R | R | S |
| 114 | H | - | R | S | S | R | S | S | C=O |
| 115 | H | - | R | R | R | R | R | R | C=O |
| 116 | Me | H | S | S | S | R | S | S | S |
| 117 | Me | H | S | R | R | R | R | R | R |
| 118 | Me | H | S | S | S | R | S | S | R |
| 119 | Me | H | S | R | R | R | R | R | S |
| 120 | Me | - | S | S | S | S | S | S | C=O |
| 121 | Me | - | S | R | R | R | R | R | C=O |
| 122 | Me | H | R | R | R | R | R | R | R |
| 123 | Me | H | R | S | S | R | S | S | S |
| 124 | Me | H | R | S | S | S | S | S | R |
| 125 | Me | H | R | R | R | S | R | R | S |
| 126 | Me | - | R | S | S | R | S | S | C=O |
| 127 | Me | - | R | R | R | R | R | R | C=O |
| 128**** | Me | H | R | R | S | R | R | S | R |

| | | | | | | | | | |
|---|---|---|---|---|---|---|---|---|---|
| * NDC-1077, when R₈ is a methyl; ** NDC-1011, when R₈ is a methyl; *** NDC-1110, when R₈ is a methyl; **** NDC-1044, when R₈ is a methyl. | | | | | | | | | |

One specific non-limiting example for treatment of an identified cancer state as described herein includes use a compound of Formula (I), which has the Formula (IX) below:

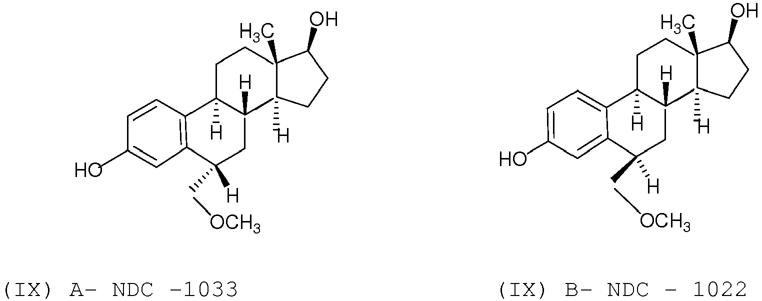

Another specific non-limiting example for treatment of an identified cancer state as described herein includes use a compound of Formula (I), which has the Formula (X), below:

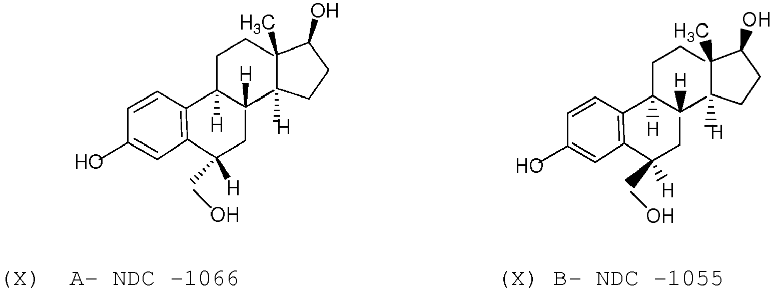

The above active compounds may also be used as part of an *in vitro* assay, for example, in order to determine whether a candidate host is likely to benefit from treatment with the compound in question. Any active compound of the present invention may also be used as a standard, for example, in an assay, in order to identify other active compounds, other anti-proliferative agents, other antiinflammatory agents, etc.

As also described herein, the candidate compounds were evaluated for their estrogen receptor antagonistic activity. The evaluation as to whether a compound is an estrogen receptor antagonist may be carried out by various methodologies known in the art. In the instant application, such capacity was determined by conducting the Luciferase binding assay according to the screening methods described herein.

In a more preferred aspect, the estrogen receptor binding capacity were assessed by transiently transfecting CV-1 cells with expression constructs for either ER(a) or ER (B) plus an ERE-tk-luciferase reporter construct. The cells were then divided into controls and candidate groups wherein the controls received no treatment, or were treated with estradiol alone (1 nM) and the candidate groups received estradiol plus an Endece compound at varying concentrations. After 16-24 hours the cells were harvested and assayed for luciferase activity using a commercially available assay kit.

As also described herein, the IC₅₀ or the half maximal inhibitory concentration of the candidate compounds were determined to assess drug potency and potential dosing regimens for in vivo use. One of ordinary skill in the art is readily able to ascertain such information using commonly known methodologies. As it has been well described in the art, IC₅₀ represents and measures how much of a particular substance/molecule is needed to inhibit some biological process by 50%. In the instant case, the IC₅₀ of the candidate compounds were determined as the concentration that led to a response of 50% compared to the vehicle control cells.

As noted herein, the salts of the compounds of this invention refer to non-toxic "pharmaceutically acceptable salts." Other salts may, however, be useful in the preparation of the compounds according to the invention or of their pharmaceutically acceptable salts. When the compounds of the present invention contain a basic group, salts encompassed within the term "pharmaceutically acceptable salts" refer to non-toxic salts which are generally prepared by reacting the free base with a suitable organic or inorganic acid. Representative salts include any such salt known in the art. Where compounds of the present invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

To treat a mammalian subject, such as a human patient, an effective amount of one or more compounds of the present invention, or a pharmaceutically-acceptable salt thereof, is administered to the mammalian subject so as to promote exposure to or contact of cancer cells or the targeted tumor growth. Effective dosage forms, modes of administration and dosage amounts may be determined empirically, and making such determinations is within the skill of the art. It is understood by the physician, veterinarian or clinician of ordinary skill in the art that the dosage amount will vary with the activity of the particular compound employed, course and/or progression of the disease state, the route of administration, the rate of excretion of the compound, renal and hepatic function of the patient, the duration of the treatment, the identity of any other drugs being administered to the subject, age, size and like factors well known in the medical arts. As discussed herein, the compounds of the present invention can be administered in such oral dosage forms as tablets, capsules (each of which includes sustained release or timed release formulations), pills, powders, micronized compositions, granules, elixirs, tinctures, suspensions, syrups and emulsions. Likewise, they may also be administered in intravenous (bolus or infusion), intraperitoneal, topical (e.g., ocular eyedrop), subcutaneous, intramuscular or transdermal (e.g., patch) form, all using forms well known to those of ordinary skill in the pharmaceutical arts. Again, the ordinarily skilled physician, veterinarian or clinician can readily determine and prescribe the effective amount of the drug required to prevent, counter or arrest the progress of the condition.

Oral dosages of the present invention, when used for the indicated effects, will range between about 0.01 mg per kg of body weight per day (mg/kg/day) to about 100 mg/kg/day, preferably 0.01 to 10 mg/kg/day, and most preferably 0.1 to 5.0 mg/kg/day. For oral administration, the compositions are preferably provided in the form of tablets containing 0.01, 0.05, 0.1, 0.5, 1.0, 2.5, 5.0, 10.0, 15.0, 25.0, 50.0, 100 and 500 milligrams of the active ingredient for the symptomatic adjustment of the dosage to the patient to be treated. A medicament typically contains from about 0.01 mg to about 500 mg of the active ingredient, preferably, from about 1 mg to about 100 mg of active ingredient. Intravenously, the most preferred doses will range from about 0.1 to about 10 mg/kg/minute during a constant rate infusion. Compounds of the present invention may be administered in a single daily dose, or the total daily dosage may be administered in divided doses of two, three or four times daily.

As noted herein, the compounds of the present invention can be used in combination with other anti-cancer agents or other agents which will enhance the treatment regime for the mammalian subject. The individual components of such combinations can be administered separately at different times during the course of therapy or concurrently in divided or single combination forms to patients or regions of such patients in need of such therapy. The instant invention is therefore to be understood as embracing all such regimes of simultaneous or alternating treatment and the term "administering" is to be interpreted accordingly. It will be understood that the scope of combinations of the compounds of this invention with other agents useful to treat the targeted cancer condition includes in principle any combination with any pharmaceutical composition useful for treating disorders related to estrogen functioning.

As used herein, the term "composition" is intended to encompass a product comprising the specified ingredients in the specified amounts, as well as any product which results, directly or indirectly, from combination of the specified ingredients in the specified amounts.

The (R) or (S)-6-methyloxoalkyl derivatives of exemestane suggest that it may be active against numerous forms of cancer beyond breast cancer.

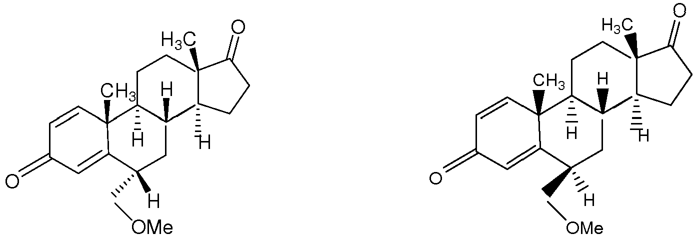

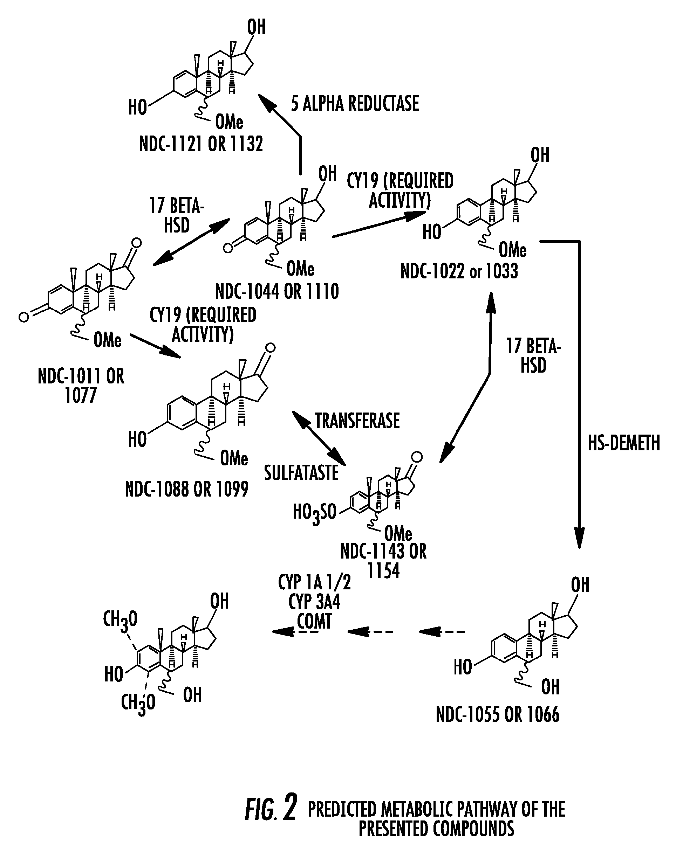

As with Estradiol, the diol compound NDC-1022 has an aromatic ring, but differs from estradiol with respect to the methyloxyalkyl substituent at the C-6 carbon. The metabolism of NDC-1011 into the diol compound NDC-1022 could occur in any order as shown in Figure 2. For example, NDC-1044 formed by 17β-HSD is converted to the NDC-1022 diol by CYP19 aromatization activity.

Without being bound to any theories, it has been reported that Estradiol binds to the receptor ligand pocket of estrogen receptors (both ERα and ERβ), via the C17-OH (via His 524); and the C3-OH (via Arg 394 and Glu 353). As with Estradiol, binding of NDC-1022 diol in the same ligand pocket of ERα and ERβ via similar amino acid bindings may occur. Additionally, the presence of the methyloxyalkyl substituent at the C-6 carbon of compound NDC-1022 may alter the conformation of the normal ligand-bound receptor resulting in modified activity accounting for the observed anti-tumor activity.

In addition, demethylase enzyme activity directed at the C-6 methyl group of NDC-1011 (or one of the metabolites of NDC-1011), may indicate the formation of triol metabolite NDC-1055. With alcohol groups at the C-3, C-6 and C-17 carbons, such an NDC-1055 triol metabolite may bind to a broad spectrum of steroid receptors in a range of tissues involving various combinations of the C-3, C-6 and C-17 alcohols. One example of such metabolites includes the compound of Formula (VII)B as shown below:

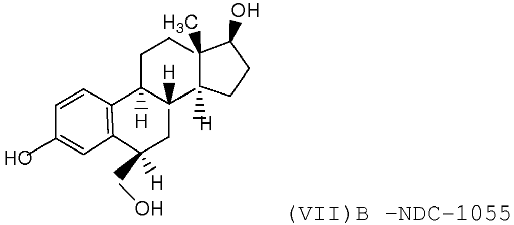

Pharmaceutical formulations of the present invention include those suitable for oral, nasal, topical (including buccal and sublingual), rectal, vaginal and/or parenteral administration. Regardless of the route of administration selected, the active ingredient(s) are formulated into pharmaceutically-acceptable dosage forms by conventional methods known to those of skill in the art.

The amount of the active ingredient (s) which will be combined with a carrier material to produce a single dosage form will vary depending upon the host being treated, the particular mode of administration and all of the other factors described above. The amount of the active ingredient(s) which will be combined with a carrier material to produce a single dosage form will generally be that amount of the active ingredient(s) which is the lowest dose effective to produce a therapeutic effect.

Methods of preparing pharmaceutical formulations or compositions include the step of bringing the active ingredient(s) into association with the carrier and, optionally, one or more accessory ingredients. In general, the formulations are prepared by uniformly and intimately bringing the active ingredient(s) into association with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or nonaqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of the active ingredient(s). The active ingredient(s) may also be administered as a bolus, electuary or paste.

In solid dosage forms of the invention for oral administration (capsules, tablets, pills, dragoes, powders, granules and the like), active ingredient(s) (in their micronized form) is/are mixed with one or more pharmaceutically-acceptable carriers, such as sodium citrate or dicalcium phosphate, and/or any of the following; (1) fillers or extenders, Such as starches, lactose, sucrose, glucose, mannitol, and/or silicic acid; (2) binders, Such as, for example, carboxymethyl-cellulose, alginates, gelatin, polyvinyl pyrrolidone, sucrose and/or acacia; (3) humectants, Such as glycerol,; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol, monostearate; (8) absorbents, such as kaolin and bentonite clay; (9) lubricants, such as talc, calcium stearate, magnesium stearate, solid polyethylene glycols, sodium lauryl sulfate, and mixtures thereof; and (10) colouring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylone glycols and the like.

A tablet may be made by compression or molding, optionally, with one or more accessory ingredients. Compressed tablets may be prepared using binder (for example, gelatin or hydroxypropylmethyl cellulose), lubricant, inert diluents, preservative, disintegrant (for example, sodium starch glycolate or cross-linked sodium carboxymethyl cellulose), surface-active or dispersing agent. Molded tablets may be made by molding in a suitable machine a mixture of the powdered active ingredient(s) moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient(s) therein using, for example, hydroxypropylmethyl cellulose in varying proportions to provide the desired release profile, other polymer matrices, liposomes and/or microspheres. They may be sterilized by, for example, filtration through a bacteria-retaining filter. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient(s) can also be in microencapsulated form.

Liquid dosage forms for oral administration of the active ingredient(s) include pharmaceutically-acceptable emulsions, microemulsions, solutions, suspensions, syrups and elixirs. In addition to the active ingredient(s), the liquid dosage forms may contain inert diluents commonly used in the art, such as, for example, water or other solvents, solubilizing agents and emulsifiers, such as ethyl alcohol, isopropyl alcohol, ethylacetate, butyl alcohol, benzyl benzoate, propylene glycol, glycol, oils (in particular, cottonseed, groundnut, corn, germ, olive, castor and sesame oils), glycerol, amyl alcohol, tetrahydrofuryl polyethylene glycols and fatty acid esters of sorbitan, and mixtures thereof.

Besides inert diluents the oral compositions can also include adjuvants such as wetting agents, emulsifying and suspending agents, sweetening, flavoring, coloring, perfuming and preservative agents. Suspensions, in addition to the active ingredient(s),may contain suspending agents as, for example, ethoxylated alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing the active ingredient(s) with one or more suitable nonirritating excipients or carriers comprising, for example, cocoa butter, polyethylene glycol, wax or salicylate and which is solid at room temperature, but liquid at body temperature and, therefore, will melt in the rectum or vaginal cavity and release the active ingredient(s). Formulations of the present invention which are suitable for vaginal administration also include pessaries, tampons, creams, gels, pastes, foams or spray formulations containing such carriers as are known in the art to be appropriate.

Dosage forms for the topical or transdermal administration of the active ingredient(s) include powders sprays, ointments, pastes, creams, lotions, gels, solutions, patches and inhalants. The active ingredient(s) may be mixed under sterile conditions with pharmaceutically-acceptable carrier, and with any buffers, or propellants which may be required.

The ointments, pastes, creams and gels may contain, in addition to the active ingredient(s), excipients, such as animal and vegetable fats, oils, waxes, paraffins, starch, tragacanth, cellulose derivatives, polyethylene glycols, silicones, bentonites, silicic acid, talc and zinc oxide, or mixtures thereof. Powders and sprays can contain, in addition to the active ingredient(s), excipients such as lactose, talc, silicic acid, aluminum hydroxide, calcium silicates and polyamide powder, or mixtures of these substances. Sprays can additionally contain customary propellants such as chlorofluorohydrocarbons and volatile unsubstituted hydrocarbons, such as butane and propane.

Compounds of the present invention may be administered in intranasal form via topical use of suitable intranasal vehicles, or via transdermal routes, using those forms of transdermal skin patches well known to those of ordinary skill in the art. A transdermal delivery system provides for continuous administration throughout the dosage regimen. Transdermal patches have the added advantage of providing controlled delivery of the active ingredient(s) to the body. Such dosage forms can be made by dissolving, dispersing or otherwise incorporating the active ingredient(s) in a proper medium, such as an elastomeric matrix material. Absorption enhancers can also be used to increase the flux of the active ingredient(s) across the skin. The rate of such flux can be controlled by either providing a rate-controlling membrane or dispersing the active ingredient(s) in a polymer matrix or gel.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Another mode of delivery for the compounds of the present invention may be delivery via the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamide-phenol, polyhydroxy-ethylaspartamide-phenol, or polyethyleneoxide-polylysine substituted with palmitoyl residues. Furthermore, the compounds of the present invention may be coupled to a class of biodegradable polymers useful in achieving controlled release of a drug, for example, polylactic acid, polyglycolic acid, copolymers of polyactic and polyglycolic acid, polyepsilon caprolactone, polyhydroxy butyric acid, polyorthoesters, polyacetals, polydihydropyrans, polycyanoacrylates and crosslinked or amphipathic block copolymers of hydrogels.

Pharmaceutical compositions of this invention suitable for parenteral administration comprise the active ingredient(s) in combination with one or more pharmaceutically-acceptable sterile isotonic aqueous or nonaqueous solutions, suspensions or emulsions, or sterile powders which may be reconstituted into sterile injectable solutions or dispersions just prior to use, which may contain antioxidants, buffers, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size, and by the use of surfactants.

These compositions may also contain adjuvants such as wetting agents, emulsifying agents and dispersing agents. It may also be desirable to include isotonic agents, such as sugars, sodium chloride, and the like in the compositions. In addition, prolonged absorption of the injectable pharmaceutical form may be brought about by the inclusion of agents which delay absorption such as aluminum monostearate and gelatin.

In some cases, in order to prolong the effect of the active ingredient(s), it is desirable to slow the absorption of the drug from subcutaneous or intramuscular injection. This may be accomplished by the use of a liquid suspension of crystalline or amorphous material having poor water solubility. The rate of absorption of the active ingredient(s) then depends upon its/their rate of dissolution which, in turn, may depend upon crystal size and crystalline form. Alternatively, delayed absorption of parenterally-administered active ingredient(s) is accomplished by dissolving or suspending the active ingredient(s)in an oil vehicle.

Injectable depot forms are made by forming microencapsule matrices of the active ingredient(s) in biodegradable polymers such as polylactide-polyglycolide. Depending on the ratio of the active ingredient(s) to polymer, and the nature of the particular polymer employed, the rate of release of the active ingredient(s) can be controlled. Examples of other biodegradable polymers include poly(orthoesters) and poly(anhydrides). Depot injectable formulations are also prepared by entrapping the active ingredient(s) in liposomes or microemulsions which are compatible with body tissue. The injectable materials can be sterilized for example, by filtration through a bacterial-retaining filter.

The formulations may be presented in unit-dose or multi-dose sealed containers, for example, ampoules and vials, and may be stored in a lyophilized condition requiring only the addition of the sterile liquid carrier, for example water for injection, immediately prior to use. Extemporaneous injection solutions and suspensions maybe prepared from sterile powders, granules and tablets of the type described above.

The pharmaceutical compositions of the present invention may also be used in the form of veterinary formulations, including those adapted for the following: (1) oral administration, for example, drenches (aqueous or nonaqueous solutions or suspensions), tablets, boluses, powders, granules or pellets for admixture with feed stuffs, pastes for application to the tongue; (2) parenteral administration, for "ampule, by subcutaneous, intramuscular or intravenous injection as, for example, a sterile solution or suspension or, when appropriate, by intramammary injection where a suspension or solution is introduced into the udder of the animal via its teat; (3) topical application, for example, as a cream, ointment or spray applied to the skin; or (4) intravaginally, for example, as a pessary, cream or foam or any other methods fit to by those of ordinary skill in the art for administration to a region of interest.

Although the present invention has been described with reference to certain embodiments, one skilled in the art will appreciate that the present invention can be practiced by other than the described embodiments, which have been presented for purposes of illustration and not of limitation. Therefore, the scope of the appended claims should not be limited to the description of the embodiments contained herein.

The general methods given in Schemes 1 and 2 for the preparation of compounds exemplified in Tables I and II above are further illustrated by the following examples. Specifically, the methods given in Schemes 1 and 2 for the preparation of 6-alkoxyalkyl Estradiol compounds are illustrated by Examples 1-5 shown below. An example of assessing the estrogen receptor binding capacity is articulated in example 4, and finally assessing the IC₅₀ of the preferred compounds of the instant invention and their comparative efficacy is given in example 5. Unless otherwise specified all starting materials and reagents are of standard commercial grade, and are used without further purification, or are readily prepared from such materials by routine methods. Those skilled in the art of organic synthesis will recognize that starting materials and reaction conditions may be varied to achieve the desired end product.

### Example 1 (Comparative)

Methods of Preparing 6-hydroxymethyl-androsta-1,4-diene-3,17 dione.

In a reaction system, sufficient amounts of (+)androsta-1,4-diene-3,17-dione (ADD), 12.2 equivalents pyrrolidine, catalytic acietic acid, denatured ethanol (95/5 ethanol/methanol) and 6-7% tetrahydrofuran (THF) were heated to 30 to 40° C for a minimum of 16 hours to form 1,3 - dipyrrolidinoandrosta-3,5-diene-17one. Once the ADD content reaches to a less than 3% by HPLC area, or it becomes static or the resulting dipyrrolidinoandrostadiene begins to revert to ADD, the reaction mixture is cooled to 5 ± 5° C. The resulting compound is then collected and washed with cold denatured ethanol. Yields are typically 70-80% on a dry basis with purities typically 90-95% by HPLC area percent.

The resulting 1,3-dipyrrolidinoandrosta-3,5-diene-17one is then mixed in amount of 1 equivalent, with 2.6 equivalents formalin (formaldehyde) in 10 ml dichloromethane/g at room temperature. The reaction mixture is then acidified to a pH of about 2 with 2% sulfuric acid solution. Accordingly, an organic layer is formed, which is washed with 2% sulfuric acid and 1:1 water/brine. Solvent exchange into toluene (approximately 10ml/g) is then carried out wherein the product crystallizes as toluene exchange traspires. Said product is collected washed and dried to provide 6-hydroxymethyl-androsta-1,4-diene-3,17 dione. One of ordinary skill in the art can further modify the stereochemistry at position 6, if so desired by employing known techniques in the art.

### Example 2

### Methods of preparing compound NDC-1022 and NDC-1033

As outlined in Scheme 2, estradiol derivatives NDC-1022, NDC-1033 were synthesized in the following manner. The protected estradiol compound 2 is prepared by reaction of compound 1 with dihydropyran in THF, using toluenesulfonic acid or camphorsulfonic acid as catalyst. As one of ordinary skill in the art can appreciate this reaction is an equilibrium reaction and would not go to completion under such conditions. Thus, both the mono-protected estradiols can be found in the reaction mixture. Such crude reaction mixture would undergo a trituration step with acetonitrile causing the desired bis-THP estradiol to crystallize in approximately 70 % yield.

As shown in Scheme 2, the key intermediate compound 3 is obtained via acylation at the benzylic 6-position with the strong base mixture referred to as LiDAKOR: butyl lithium, diisopropylamine, and potassium tert-amylate. Under such conditions at -70 °C, one of ordinary skill in the art can appreciate the abstraction of a proton at a benzylic position. The intermediate compound 3 is then purified by column chromatography to give a syrup in approximately 50 % yield, still containing minor impurities and column solvents. Reduction of the aldehyde with an excess of lithium aluminum hydride results in high yields of the racemic hydroxymethyl estradiol compound 4 as a glassy foam.

For purposes of preparing NDC-1022 and NDC 1033, the methoxymethyl intermediate compound 7 was prepared by methylation of compound 4 with sodium hydride and methyl iodide. Compound 7 was purified by column chromatography to give a glassy foam. Deprotecting the protected groups would give racemic 6-methoxymethyl estradiol compound 8. Separation of the enantiomers was performed using chiral preparative HPLC to give the compounds NDC-1022 and NDC-1033. For compound NDC-1022, a chiral purity of >95:5 5 *R:S* was realized. For compound NDC-1033, a chiral purity of 86:14 *S:R* was realized. It is well within the level of one of ordinary skill in the art to employ NMR for determination of the absolute stereochemistry of the 6-position, where the 4-and 6-protons are diagnostic.

### Example 3

### Methods of preparing NDC-1055 and NDC-1066

Using the same methodologies described in Example 2, compound 4 is synthesized. Deprotection of compound 4 was then achieved with catalytic hydrogen chloride in methanol, and racemic compound 5 was separated on chiral preparative HPLC to give two fractions, one enriched for NDC-1055 and the other enriched for NDC-1066. For each compound, chiral purity of >95:5 *R:S* and *S:R* was realized respectively. Absolute stereochemistry of the 6-position was established by NMR, where the 4-and 6-protons are diagnostic.

### Example 4

Methods of determining estrogen receptor binding capacity using Luciferase activity.

Estrogen receptor-negative CV-1 kidney cells were maintained in Dulbecco's modified Eagle's medium with 4.5 g/L glucose supplemented with 10% fetal bovine serum and 100 units / ml penicillin-streptomycin at 37° C in a humidified 5% CO₂ atmosphere.

The cells were then plated in 6-well dishes at a density of 2 x 10⁵ cells per well in phenol-red free Dulbecco's modified Eagle's medium containing 10% charcoal-dextran-stripped fetal bovine serum. CV-1 cells were transfected using LipofectAMINE reagent according to the manufacturer's protocol. Transfections containing 1.5 ug of reporter plasmid (containing ERE-tk-luciferase containing a single ERE cloned upstream of the thymidine kinase promoter and luciferase gene) and 0.5 ug of either ERa or ERß expression vector (containing CMV-ERa or CMV-ER_{ß} full length coding sequence respectively).

The next day, cells received no treatment (controls) or were treated with estradiol alone (1 nM) or estradiol plus an Endece compound (at varying concentrations). After 16-24 hours, cells were harvested and assayed for luciferase activity.

At the outset, cell monolayers were washed twice with ice-cold phosphate-buffered saline and incubated for 15 minutes in 250 ul of 1X cell culture lysis reagent (Promega, Madison, WI). Cell extracts were transferred to a fresh tube and assayed using the luciferase assay system (Promega). For each assay, 10 ul of extract was diluted with 90 ul of 1X cell culture lysis reagent. Luminescence was read using an AutoLumat LB953 luminometer.

A compound or a salt thereof, which is identified by the binding assay described herein is a compound that inhibits the binding of estrodial at the ligand binding site of the estrogen receptors. Specifically, it is a compound or a salt thereof that is envisioned to cause cell proliferation statasis and accordingly exerts its pharmacological activity. As indicated in Figure 3 and 4, lead compounds NDC-1022, NDC-1033 exhibit strong competitive behavior against estradiol in binding to either of the estrogen receptors thereby causing stasis of cell proliferation activity.

### Example 5

Method of determining the IC₅₀ values of the candidate compounds. The cell lines listed were maintained at approximately 5% CO₂, 37°C, 95% relative humidity in the media appropriate for that cell line. The cells were subcultured every two to three days and plated in clear bottom 96-well plates at a density of 1 x 10⁴ cells/well and incubated at ca. 5% CO₂, 37°C overnight prior to initiation of the assay. To begin cell viability assays, the media in the cell plate (100 µL) was replaced with fresh media (100 µL). The test articles were serially diluted 1:2 in fresh media in duplicate and added to the cells (100 µL) at final sample concentrations of 0.46, 1.37, 4.12, 12.35, 37.04, 111.1, 333.3 and 1000 µM (≤ 1% DMSO) in a total volume of 200 µL. Wells containing no cells and wells containing cells lysed with 0.1% Triton-X were used for baseline controls. Tamoxifen was used as a known control for each assay and DMSO only will be run as vehicle control. The samples were incubated at *ca.* 37 °C in humidified 5% CO₂ atmosphere for 72 hours. The plate was monitored once a day during the incubation period, paying special attention to the level of confluence. If the cells approach confluence prior to the end of the 72 hour incubation period the experiment was terminated at that time and cell viability measured as described below.

Cell Viability was determined using a commercially available kit to determine ATP levels by luminescence. Briefly, the cell plate had the media removed and replaced with 100 µL of fresh media, and the buffer and lyophilized substrate were equilibrated to room temperature. The buffer was used to reconstitute the substrate just prior to addition to the wells of the cell plate (100 µL per well). The plate was placed into the Infinite M200 plate reader, allowed to shake for 10 minutes followed by a 10 minute wait period, the plate was read using an integration time of 0.5 sec with no attenuation.

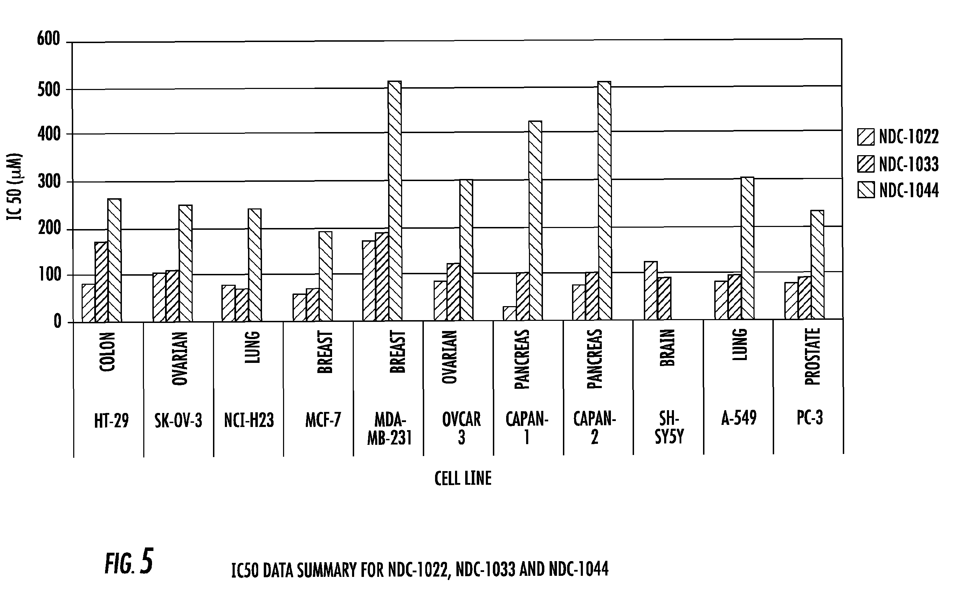

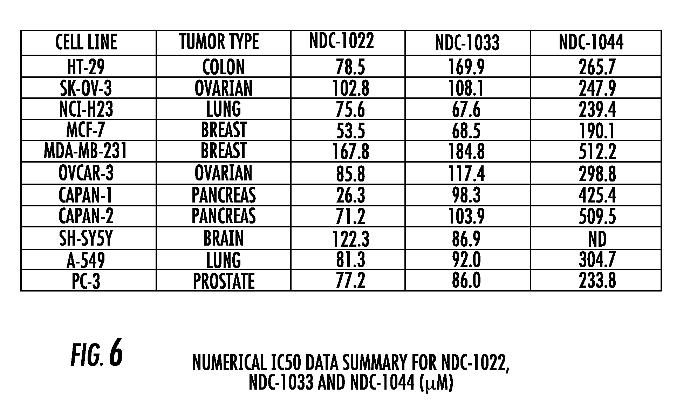

The mean baseline controls (wells with Triton X-100 or no cells) were subtracted from the total luminescence to give the net luminescence for that well. This total was compared to the control of DMSO only. An IC₅₀ was calculated as the concentration that led to a response of 50% compared to the vehicle control cells. Figures 5 and 6 depict the results of the tests. Accordingly, those of ordinary skill in the art can appreciate that the R configuration of the instantly claimed composition are superior to other stereoisomers.

## Claims

1. A compound having the structure of Formula (I): 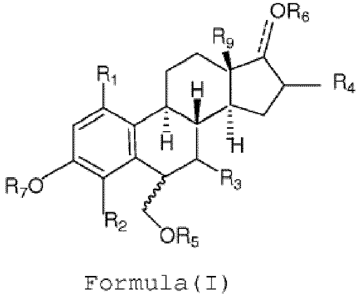
wherein R₁, R₂, R₃, R₄, R₆, R₇ and R₉ are independently hydrogen, C₁ to C₆ alkyl or substituted alkyl, halogen, sulfate or glucuronide moieties;
R₅ is C₁ to C₆ alkyl or substituted alkyl,
the ---- symbol represents either a single or a double bond and when the ---- symbol is a double bond at position 17 it forms a keto group; and the 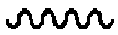 symbol represents any type of bond regardless of the stereochemistry; and the enantiomers, other stereochemical isomers, hydrates, solvates, tautomers and pharmaceutically acceptable salts of said compounds.

2. The compound of Formula (I) of claim 1, wherein R₁, R₂, R₃, R₄, R₆, and R₇ are independently hydrogen, methyl, or ethyl.

3. The compound of any one of claims 1 -2, wherein the 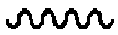 symbol represents a
bond forming a R or a S stereoisomer.

4. A compound according to Formula (I) of claim 1 having the structure: 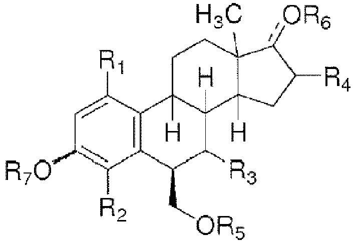 wherein:
R₁, R₂, R₃, R₄ are independently selected from the group consisting of H, C₁-C₆ alkyl, substituted alkyl, and halogen;
R₅ is C₁-C₆ alkyl,
R₆, R₇ are independently selected from the group consisting of H, C₁-C₆ alkyl, and
the ---- symbol represents either a single or a double bond at position 17, with the proviso that when ---- symbol is a double bond it forms a keto group at position 17 and no R₆ is present.

5. The compound of claim 4 wherein R₆ and R₇ are independently selected from a group consisting of methyl and hydrogen.

6. The compound of claims 1 or 4 wherein R₅ is methyl and R₁, R₂, R₃, R₄ are
hydrogens.

7. A compound of any of the preceding claims, having the formula selected from the group consisting of:

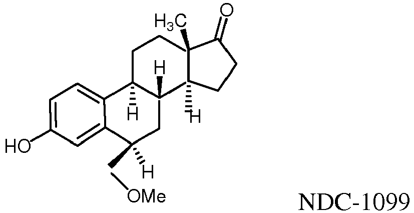

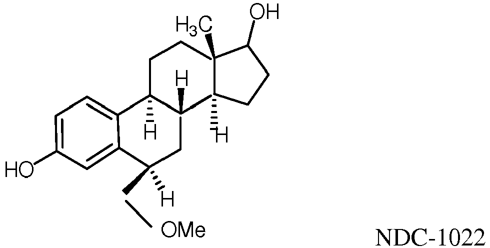

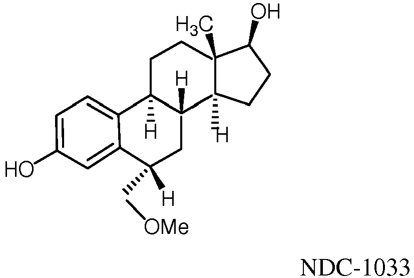

8. The compound of claim 1, wherein the carbon at position 6 is an asymmetric centre that has an R configuration.

9. A pharmaceutical composition comprising a compound of claim 1 or 4 and a
pharmaceutically-acceptable carrier.

10. A pharmaceutical composition of claim 9 wherein the compound has an
asymmetric centre at position 6 that has an R configuration.

11. A pharmaceutical composition according to claim 10 wherein the compound
occurs substantially free of other isomers.

12. A pharmaceutical composition of claim 9 or 10 wherein the compound occurs as
a racemic mixture, or as individual diastereomers or enantiomers.

13. A compound of any one of claims 1 -8 or composition of any one of claims 9-12,
for use in inhibiting growth of cancer cells of a solid tumor selected from the group consisting of breast, pancreatic, lung, colon, prostate, ovarian, brain, liver, spleen, kidney, lymph node, small intestine, blood cells, bone, stomach, endometrium, testicular, ovary, central nervous system, skin, head and neck, esophagus, and bone marrow cancer.

## Patentansprüche

1. Verbindung mit einer Struktur der Formel (I): 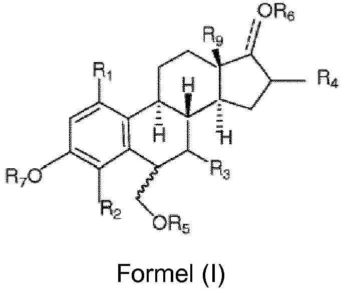
worin R₁, R₂, R₃, R₄, R₆, R₇ und R₉ jeweils unabhängig Wasserstoff, C₁₋₆-Alkyl oder substituierte Alkyl-, Halogen-, Sulfat- oder Glucuronid-Gruppierungen sind;
R₅ C₁₋₆-Alkyl oder substituiertes Alkyl ist,
das Symbol ---- entweder für eine Einfach- oder für eine Doppelbindung steht und, wenn das Symbol ---- eine Doppelbindung an Position 17 ist, eine Ketogruppe bildet, und das Symbol 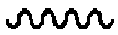 für jedwede Art von Bindung ungeachtet der Stereo-chemie steht; sowie Enantiomere, sonstige stereochemische Isomere, Hydrate, Solvate, Tautomere und pharmazeutisch annehmbare Salze dieser Verbindung.

2. Verbindung der Formel (I) nach Anspruch 1, worin R₁, R₂, R₃, R₄, R₆ und R₇ jeweils unabhängig Wasserstoff, Methyl oder Ethyl sind.

3. Verbindung nach einem der Ansprüche 1 bis 2, worin das Symbol 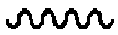 für eine Bindung steht, die ein R- oder ein S-Stereoisomer bildet.

4. Verbindung nach Formel (I) nach Anspruch 1 mit der folgenden Struktur: 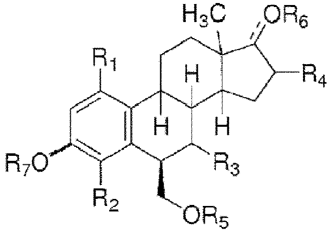 worin:
R₁, R₂, R₃, R₄ jeweils unabhängig aus der aus H, C₁₋₆-Alkyl, substituiertem Alkyl und Halogen bestehenden Gruppe ausgewählt sind;
R₅ C₁₋₆-Alkyl ist,
R₆, R₇ jeweils unabhängig aus der aus H, C₁₋₆-Alkyl bestehenden Gruppe ausgewählt sind und
das Symbol ---- entweder für eine Einfach- oder für eine Doppelbindung an Position 17 steht, mit der Maßgabe, dass, wenn das Symbol ---- eine Doppelbindung ist, diese eine Ketogruppe an Position 17 bildet und kein R₆ vorhanden ist.

5. Verbindung nach Anspruch 4, worin R₆ und R₇ jeweils unabhängig aus einer aus Methyl und Wasserstoff bestehenden Gruppe ausgewählt sind.

6. Verbindung nach Anspruch 1 oder 4, worin R₅ Methyl ist und R₁, R₂, R₃, R₄ jeweils Wasserstoff sind.

7. Verbindung nach einem der vorangegangenen Ansprüche mit der aus der aus Folgendem bestehenden Gruppe ausgewählten Formel:

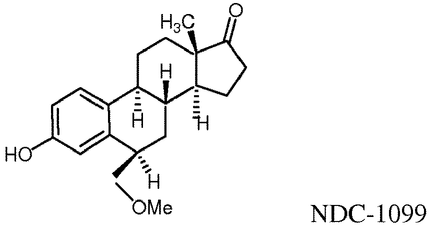

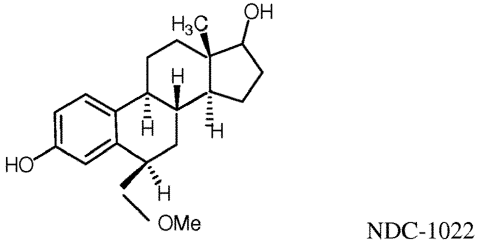

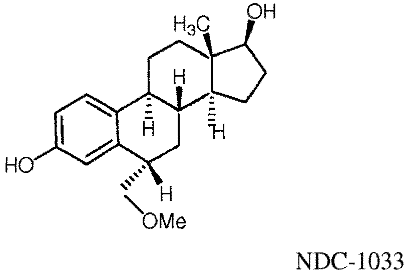

8. Verbindung nach Anspruch 1, worin der Kohlenstoff an Position 6 ein asymmetrisches Zentrum ist, das R-Konfiguration aufweist.

9. Pharmazeutische Zusammensetzung, die eine Verbindung nach Anspruch 1 oder 4 und einen pharmazeutisch annehmbaren Träger umfasst.

10. Pharmazeutische Zusammensetzung nach Anspruch 9, worin die Verbindung ein asymmetrisches Zentrum an Position 6 mit R-Konfiguration aufweist.

11. Pharmazeutische Zusammensetzung nach Anspruch 10, worin die Verbindung frei von sonstigen Isomeren vorliegt.

12. Pharmazeutische Zusammensetzung nach Anspruch 9, worin die Verbindung als racemisches Gemisch oder als einzelne Diastereomere oder Enantiomere vorliegt.

13. Verbindung nach einem der Ansprüche 1 bis 8 oder Zusammensetzung nach einem der Ansprüche 9 bis 12 zur Verwendung zur Hemmung der Vermehrung von Krebszellen eines aus der aus Brust-, Bauchspeicheldrüsen-, Lungen-, Dickdarm-, Prostata-, Eierstock-, Hirn-, Leber-, Milz-, Nieren-, Lymphknoten-, Dünndarm-, Blutzellen-, Knochen-, Magen-, Endometrium-, Hoden-, Eierstock-, Zentralnervensystem-, Haut-, Kopf-Hals-, Speiseröhren- und Knochenmarkskrebs bestehenden Gruppe ausgewählten soliden Tumors.

## Revendications

1. Composé ayant une structure de formule (I) : 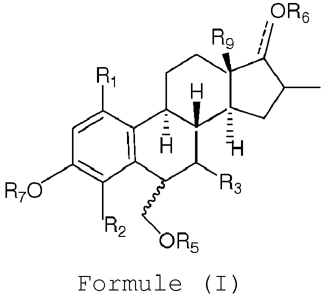 dans laquelle
R₁, R₂, R₃, R₄, R₆, R₇ et R₈ sont indépendamment l'hydrogène, un alkyle ou alkyle substitué en C₁ à C₆, un halogène, un sulfate ou un fragment glucuronide ;
R₅ est un alkyle ou alkyle substitué en C₁ à C₆,
le symbole ---- représente une liaison soit simple soit double, et quand le symbole ---- est une double liaison en position 17, il forme un groupe céto ; et le symbole 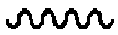 représente n'importe quel type de liaison quelle que soit la stéréochimie ;
et les énantiomères, autres isomères stéréochimiques, hydrates, solvates, tautomères et sels pharmaceutiquement acceptables desdits composés.

2. Composé de formule (I) selon la revendication 1, dans lequel R₁, R₂, R₃, R₄, R₆ et R₇ sont indépendamment l'hydrogène, méthyle, ou éthyle.

3. Composé selon l'une quelconque des revendications 1 et 2, dans lequel le symbole 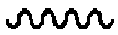 représente une liaison formant un stéréoisomère R ou S.

4. Composé de formule (I) selon la revendication 1, ayant la structure : 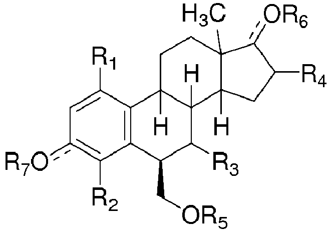 dans laquelle
R₁, R₂, R₃ et R₄ sont indépendamment choisis dans l'ensemble constitué par H, alkyle en C₁ à C₆, alkyle substitué, et les halogènes ;
R₅ est un alkyle en C₁ à C₆,
R₆ et R₇ sont indépendamment choisis dans l'ensemble constitué par H et alkyle en C₁ à C₆, et
le symbole ---- représente une liaison soit simple soit double en position 17, sous réserve que, lorsque le symbole ---- est une double liaison, il forme un groupe céto en position 17 et aucun R₆ ne soit présent.

5. Composé selon la revendication 4, dans lequel R₆ et R₇ sont indépendamment choisis dans l'ensemble constitué par méthyle et l'hydrogène.

6. Composé selon la revendication 1 ou 4, dans lequel R₅ est méthyle et R₁, R₂, R₃ et R₄ sont des hydrogènes.

7. Composé selon l'une quelconque des revendications précédentes, de formule choisie dans l'ensemble constitué par :

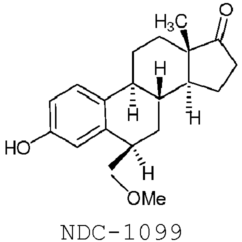

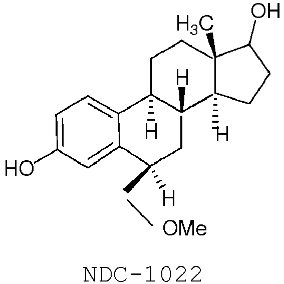

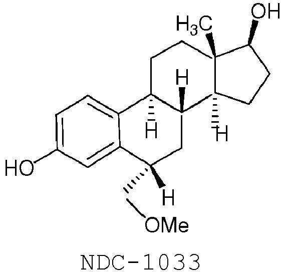

8. Composé selon la revendication 1, dans lequel le carbone en position 6 est un centre asymétrique qui a une configuration R.

9. Composition pharmaceutique comprenant un composé selon la revendication 1 ou 4 et un véhicule pharmaceutiquement acceptable.

10. Composition pharmaceutique selon la revendication 9, dans lequel le composé a un centre asymétrique en position 6 qui a une configuration R.

11. Composition pharmaceutique selon la revendication 10, dans laquelle le composé apparaît en étant pratiquement exempt d'autres isomères.

12. Composition pharmaceutique selon la revendication 9, dans lequel le composé apparaît sous la forme d'un mélange racémique, ou sous la forme de diastéréomères ou énantiomères individuels.

13. Composé selon l'une quelconque des revendications 1 à 8 ou composition selon l'une quelconque des revendications 9 à 12, pour utilisation dans l'inhibition de la croissance de cellules cancéreuses d'une tumeur solide choisie dans l'ensemble constitué par les cancers du sein, du pancréas, du poumon, du côlon, de la prostate, de l'ovaire, du cerveau, du foie, de la rate, du rein, des ganglions lymphatiques, de l'intestin grêle, des cellules sanguines, des os, de l'estomac, de l'endomètre, des testicules, de l'ovaire, du système nerveux central, de la peau, de la tête et du cou, de l'oesophage, et de la moelle osseuse.
